# EUROPÄISCHE PATENTANMELDUNG

(11) **EP 1 714 671 A1**
(43) Veröffentlichungstag der Anmeldung: **25.10.2006**
(21) Anmeldenummer: 05008630.5
(22) Anmeldetag: 20.04.2005
(51) Int. Cl.: A61N 1/04, A61N 1/39

(54) **Defibrillierelektrode**

(71) Anmelder: Elovis GmbH, 76133 Karlsruhe (DE)
(72) Erfinder: Stork, Wilhelm, Dr., 76831 Impflingen (DE); Wagner, Armin, Dipl.Phys., 76131 Karlsruhe (DE); Hey, Stefan, 76831 Impflingen (DE)
(74) Vertreter: Späth, Dieter

(57) **Zusammenfassung**

Die Erfindung betrifft eine permanent tragbare Defibrillierelektrode (1, 4) mit einer Wabenstruktur (3) als Reservoir (2) für ein Leitmedium, insbesondere ein Leitgel (6), das im Bedarfsfall mittels eines Gaserzeugers (12) durch die Defibrillierelektrode (4) durchgepresst wird. Der Gaserzeuger (12) kann eine elektrische Widerstandsheizung (10) und eine Chemikalie (11) bzw. ein Chernikaliengemisch aufweisen, das durch Wärmeeinwirkung chemisch reagiert und ein Gas zur Druckerzeugung bildet.

## Beschreibung

Die Erfindung betrifft eine Defibrillierelektrode zum Tragen auf der Haut mit den Merkmalen des Oberbegriffs des Anspruchs 1.

Nach einem Herzinfarkt oder einer Herzoperation erleidet ein großer Prozentsatz der Patienten innerhalb von sechs Monaten ein Herzkammerflimmern. Lebensrettend ist ausschließlich ein schnelles Erkennen des Herzkammerflimmerns und eine Defibrillation, d. h. ein Stromschock mittels zwei Elektroden im Brustbereich. Der Stromschock bringt, sofern die Defibrillation erfolgreich ist, das Herz wieder zum Schlagen. Wünschenswert wäre deswegen ein mobiler, vom Patienten während einer Risikozeit von beispielsweise sechs Monaten permanent zu tragender Defibrillator, der das Herzkammerflimmem erkennt und die Defibrillation selbsttätig durchführt. Dazu sind zwei dauerhaft im Brustbereich des Patienten angebrachte Defibrillierelektroden notwendig.

Zum Defibrillieren ist ein elektrisch leitendes Medium (Leitmedium) notwendig, das zwischen der Haut des Patienten und den Defibrillierelektroden aufgetragen ist und das einen Stromübergangswiderstand zwischen den Defibrillierelektroden und der Haut herabsetzt, weil ansonsten die Stromstärke des zur Defibrillation erzeugten Stromschocks die Haut verbrennt, u. U. mit der Folge, dass der auf das Herz einwirkende Stromschock im Körper zu schwach für die Defibrillation ist.

Als Leitmedium wird üblicherweise ein Leitgel verwendet. Die Verwendung eine anderen Leitmediums ist denkbar und nicht von der Erfindung ausgeschlossen. So ist beispielsweise auch an ein pulverförmiges Leitmedium zu denken, das mit der Feuchtigkeit der Haut ein fließfähiges und sich zwischen der Defibrillierelektrode und der Haut verteilendes Leitmedium bildet.

Ein dauerhaftes Auftragen des Leitmediums ist jedoch aus mehreren Gründen nicht möglich: Zum einen bewirkt das dauerhaft aufgetragene Leitmedium eine Hautreizung mit einem für den Patienten nicht auszuhaltenden Juckreiz und ist deswegen nicht akzeptabel. Zum anderen trocknet ein üblicherweise als Leitmedium verwendetes Leitgel aus, verflüchtigt sich und/oder wird u. U. von der Haut aufgenommen, d. h. nach wenigen Stunden ist kein Leitmedium ausreichender elektrischer Leitfähigkeit mehr vorhanden. Einziger Ausweg ist derzeit die Implantation von Defibrillierelektroden unter die Haut.

Der Erfindung liegt daher die Aufgabe zu Grunde, eine Defibrillierelektrode mit gutem Tragekomfort vorzuschlagen.

Diese Aufgabe wird erfindungsgemäß durch die Merkmale des Anspruchs 1 gelöst. Der Erfindung liegt die Idee zu Grunde, das zur Defibrillation notwendige Leitmedium in der Defibrillierelektrode zu speichern und im Bedarfsfall auf der hautzugewandten Seite aus der Defibrillierelektrode auszupressen. Dazu ist die erfindungsgemäße Defibrillierelektrode durchlässig für ein Leitmedium, das in einem Reservoir auf einer hautabgewandten Seite der Elektrode enthalten ist. Dabei bezeichnet hautabgewandt die Seite der Defibrillierelektrode, auf der das Leitmedium aufbewahrt ist und eine gegenüberliegende, hautzugewandte Seite der Defibrillierelektrode ist zum Aufsetzen auf die Haut des Patienten vorgesehen. Des Weiteren weist die erfindungsgemäße Defibrillierelektrode eine Einrichtung zum Durchdrücken des Leitmediums durch die Defibrillierelektrode zu ihrer Aktivierung auf. Die Einrichtung zum Durchdrücken des Leitmediums wird auch nachfolgend als Aktivierungseinrichtung bezeichnet werden. Die Defibrillierelektrode kann immer durchlässig für das Leitmedium sein oder bei der Aktivierung durchlässig für das Leitmedium werden. Als Leitmedium ist insbesondere ein Leitgel vorgesehen, wie es zur Defibrillation bekannt ist. Die Verwendung eines anderen Leitmediums, beispielsweise auch eines Pulvers, das evtl. erst mit der Feuchtigkeit der Haut eines Patienten ein fliessfähiges und sich zwischen der Defibrillierelektrode und der Haut verteilendes Medium bildet, ist denkbar und möglich.

Die Defibrillierelektrode ist elektrisch leitend und vorzugsweise flexibel, sie kann ein Metallgewebe, eine perforierte Metallfolie, eine Metall-/ Kunststoffverbundfolie, eine Folie aus leitfähigem Kunststoff, beispielsweise durch eingelagerte Metallpartikel, oder eine metallisierte Folie aufweisen. Mit der Defibrillierelektrode wird der Herzschlag gemessen bzw. überwacht, um einen Herzstillstand oder ein Herzkammerflimmem zu erkennen und die Defibrillation einzuleiten. Der Strom bei der Messung des Herzschlags ist gering und problemlos ohne Leitmedium messbar. Die erfindungsgemäße Defibrillierelektrode lässt sich deswegen ohne Leitmedium und somit dauerhaft tragen. Dabei ist an eine Tragedauer der Defibrillierelektrode von beispielsweise etwa sieben Tagen gedacht, danach werden die Defibrillierelektroden gewechselt. Im Falle eines Herzstillstands oder eines Herzkammerflimmerns wird die Defibrillierelektrode durch Durchdrücken des Leitmediums durch die Defibrillierelektrode mittels der Aktivierungseinrichtung aktiviert, das Leitmedium gelangt aus dem Reservoir zwischen die Defibrillierelektrode und die Haut. Das Durchdrücken des Leitmediums dauert zwischen weniger als 1 Sekunde bis zu einigen Sekunden. Anschließend erfolgt die Defibrillation durch Stromschock, der, sofern nicht erfolgreich, wiederholt und dessen Stärke gesteigert werden kann.

Vorteil der erfindungsgemäßen Defibrillierelektrode ist deren guter Tragekomfort, da sie ohne Leitmedium auf der Haut und in Folge dessen dauerhaft getragen werden kann. Des Weiteren ist das Aufsetzen der Defibrillierelektrode auf die Haut einfach und auch von einem Laien, beispielsweise dem Patienten selbst, problemlos und zuverlässig zu bewerkstelligen. Die Erfindung ermöglicht den Einsatz eines mobilen, von einem Patienten permanent tragbaren Defibrillators, der die Herztätigkeit überwacht und erforderüchenfalls die Defibrillation vornimmt.

In bevorzugter Ausgestaltung der Erfindung weist die Aktivierungseinrichtung eine Druckerzeugungseinrichtung auf, die bei einer Aktivierung im Reservoir einen Druck erzeugt, der das Leitmedium durch die Defibrillierelektrode durchdrückt.

Eine Ausgestaltung der Erfindung sieht eine Gasdruckerzeugungseinrichtung vor. Die Gaserzeugung kann chemisch, beispielsweise durch eine sich unter Gasbildung zersetzende Chemikalie oder ein Chemikaliengemisch, oder durch Reaktion von Chemikalien miteinander, wobei sich ein Gas- oder Gasgemisch bildet, erfolgen. Derartige Chemikalien und chemische Reaktionen sind an sich bekannt und brauchen deswegen an dieser Stelle nicht näher erläutert zu werden. Als Beispiel sei die Reaktion von Ammoniumhydrogencarbonat zu Ammoniak, Kohlendioxid und Wasser genannt (NH₄HCO₃ → CO₂ + H₂O + NH₃). Als weitere Ausgangsstoffe für eine chemische Reaktion zur Druckgaserzeugung zum Verdrängen des Leitmediums aus dem Reservoir durch die Defibrillierelektrode sind Ammoniumsalze, Carbonate, Nitrite und andere mehr möglich. Ausgelöst wird die chemische Reaktion beispielsweise durch Erwärmen, wobei bei einer exothermen chemischen Reaktion das Erwärmen nur zum Einleiten der chemischen Reaktion erforderlich ist, wogegen bei einer endothermen chemischen Reaktion beständig Wärme zugeführt werden muss. Die Erwärmung kann durch eine Widerstandsheizung ertolgen. Die chemische Reaktion kann auch durch Kontakt der Chemikalien erfolgen, indem beispielsweise eine die Chemikalien trennende Membran geschmolzen wird. Auch eine Verbrennung, also eine Oxidation oder eine katalytische Reaktion eignen sich zur Gas- und Druckerzeugung zum Durchdrücken des Leitmediums aus dem Reservoir durch die Defibrillierelektrode zu deren Aktivierung.

Eine Ausgestaltung der Erfindung sieht vor, dass der Gaserzeuger eine Heizung oder Zündung zum Auslösen oder zum Durchführen der chemischen Reaktion zur Gaserzeugung aufweist. Die Heizung ist insbesondere eine Widerstandsheizung, es eignen sich gedruckte Schaltungen mit dünnen, einen elektrischen Widerstand bildenden Leiterbahnen oder mit auf einer Leiterplatte aufgebrachtem Widerstandsmaterial, beispielsweise Graphit-Pastendruck. Zur Zündung können zwei oder mehr Leiterbahnen, die Zündelektroden bilden, mit Abstand voneinander enden.

Eine andere Ausgestaltung der Erfindung sieht eine elektrolytische Gaserzeugung vor. Mit Elektroden, die wiederum Leiterbahnen einer gedruckten Schaltung sein können, wird ein Elektrolyt durch Stromfluss zersetzt und Gas gebildet. Das Elektrolyt kann eventuell ein als Leitmedium verwendetes Leitgel sein.

Eine andere Ausgestaltung der Erfindung sieht einen Dampferzeuger als Aktivierungseinrichtung vor, der bei Erwärmen Dampf erzeugt. Auch bei dieser Ausgestaltung der Erfindung kann eventuell ein Teil des Leitmediums durch Erwärmen verdampft werden und der dadurch erzeugte Druck das übrige Leitmedium durch die Defibrillierelektrode durchdrücken. Ansonsten ist es möglich, ein verdampfbares festes oder flüssiges Medium in einem der Defibrillierelektrode fernen Teil des Reservoirs aufzubewahren und zur Aktivierung zu erwärmen.

Als Reservoir sieht eine Ausgestaltung der Erfindung eine Löcher aufweisende Struktur vor, in deren Löcher das Leitmedium und ggf. die bei Aktivierung gasbildenden Chemikalien, das Elektrolyt oder das verdampfbare Medium, aufbewahrt werden. Die Löcher aufweisende Struktur kann regelmäßig beispielsweise als Lochraster oder in Wabenform ausgeführt sein, sie kann auch ein offenporiger Schaum sein. Als Material eignet sich Kunststoff in Scheibenform, der auf die Defibrillierelektrode aufgebracht ist. Die Herstellung kann durch Extrudieren, Spritzgießen, Pressen, Prägen, Laserperforieren usw. erfolgen. Die Löcher aufweisende Struktur ist einfach und preisgünstig in ihrer Herstellung, ihre Löcher sind einfach mit dem Leitmedium und erforderlichenfalls weiteren Medien füllbar, sie ist einfach auf der Defibrillierelektrode anbringbar und bewahrt das Leitmedium zuverlässig, dauerhaft und gegen Austrocknen geschützt auf.

Eine Ausgestaltung der Erfindung sieht eine poröse Defibrillierelektrode vor. Durch die Porösität ist das Leitmedium durch Druckbeaufschlagung durch die Defibrillierelektrode durchdrückbar. Bei drucklosem Leitmedium hält die Defibrillierelektrode das Leitmedium im Reservoir und schützt es vor Austrocknung.

Eine andere Ausgestaltung sieht Durchlässe in der Defibrillierelektrode für das Leitmedium vor. Die Durchlässe können beispielsweise die Form von Schlitzen, beispielsweise auch einander kreuzende Schlitze, oder Löcher aufweisen, die beispielsweise durch Laserbearbeitung hergestellt sind. Die Durchlässe können offen oder geschlossen sein und reißen in letzterem Fall erst durch die Druckeinwirkung bei Aktivierung der Defibrillierelektrode auf. Geschlossene Durchlässe sind beispielsweise durch Schlitze gebildet, die nicht vollständig durch die Defibrillierelektrode durchgehen. Auch offene Durchlässe sind in drucklosem Zustand vorzugsweise geschlossen, indem die Durchlässe oder Schlitze keine lichte Weite haben, sondern ihre Wandungen aneinander anliegen. Sie öffnen sich durch Druckbeaufschlagung des Leitmediums. Ansonsten sind offene Durchlässe vorzugsweise schmal, um eine Austrocknung des Leitmediums zu vermeiden oder jedenfalls so stark zu verringern, dass nach einer Einsatzdauer der Defibrillierelektrode von beispielsweise sieben Tagen noch ausreichend Leitmedium für die Defibrillation im Reservoir enthalten ist. Auch ist die Verwendung eines Leitgels auf beispielsweise Silikon- anstatt auf Wasserbasis als Leitmedium möglich, um die Austrocknung zu vermeiden.

Eine Ausgestaltung der Erfindung sieht eine Gas- oder Dampfblase im Reservoir auf der Seite der Defibrillierelektrode vor. Die Gas- oder Dampfblase verringert einen Dampfdruckgradienten vom Leitmedium zur hautzugewandten Seite der Defibrillierelektrode und verringert dadurch die Austrocknung des Leitmediums auf einen Bruchteil. Dieses aus der Natur, nämlich bei Kakteen, abgeschaute Prinzip zur Verringerung der Verdunstung und zur Vermeidung der Austrocknung verlängert die Einsatzdauer der Defibrillierelektrode auf mehrere Wochen oder Monate.

Eine Ausgestaltung der Erfindung sieht einen Schichtaufbau (Laminat) der erfindungsgemäßen Defibrillierelektrode vor. Dabei ist auf einer Seite der Löcher aufweisenden, das Reservoir bildenden Struktur die für das Leitmedium durchlässige und elektrisch leitende Defibrillierelektrode und auf der anderen Seite der Löcher aufweisenden Struktur eine Leiterplatte mit Leiterbahnen zur Aktivierung der Defibrillierelektrode, d. h. zur Druckerzeugung und zum Durchdrücken des Leitmediums aus der Löcher aufweisenden Struktur durch die Defibrillierelektrode auf der anderen Seite der Löcher aufweisenden Struktur angebracht. Als zusätzlicher Vorteil kann auf der Leiterplatte eine Mess-, Steuer-, Regel- oder dgl. Elektronik angeordnet sein.

In bevorzugter Ausgestaltung der Erfindung ist die Defibrillierelektrode flexibel zur Anpassung an den Patienten.

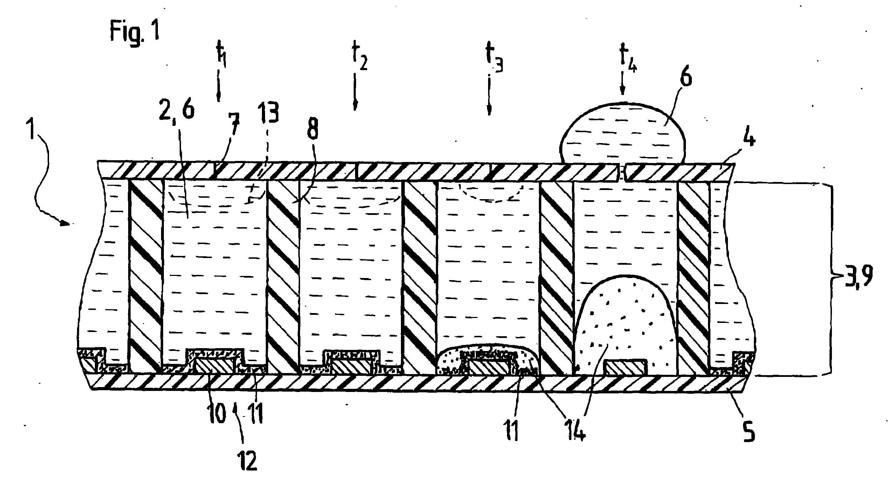

Die Erfindung wird nachfolgend anhand eines in der Zeichnung dargestellten Ausführungsbeispiels näher erläutert. Die einzige Figur zeigt einen Schnitt einer erfindungsgemäßen Defibrillierelektrode mit zeitlich aufeinander folgenden Aktivierungsschritten t₁ bis t₄. Die Zeichnung ist als vereinfachte Schemadarstellung zu verstehen.

Die in der Zeichnung dargestellte, erfindungsgemäße Defibrillierelektrode 1 weist einen Schichtaufbau (Laminat) mit einer plattenförmigen, Löcher 2 aufweisenden Struktur 3 auf, auf deren einer Seite die eigentliche Defibrillierelektrode 4 und auf deren anderer Seite eine Leiterplatte 5 angebracht sind. Die eigentliche Defibrillierelektrode 4 und die Leiterplatte 5 weisen einen Bruchteil einer Dicke der Löcher 2 aufweisenden Struktur 3 auf. Alle Schichten der Defibrillierelektrode 1 und damit die Defibrillierelektrode 1 insgesamt sind flexibel.

Die eigentliche Defibrillierelektrode 4 besteht aus einer elektrisch leitenden Folie. Zur Erzeugung der Leitfähigkeit sind beispielsweise Metallpartikel in die Folie eingelagert, es ist eine Metallschicht aufgedampft, des handelt sich um eine Kunststoff-/Metall-Verbundfolie oder um eine Metallfolie. Die eigentliche Defibrillierelektrode 4 ist durchlässig für ein als elektrisch leitendes Medium (Leitmedium) verwendetes Leitgel 6, wenn das Leitgel 6 durch die Defibrillierelektrode durchgedrückt wird. Drucklos erfolgt kein Durchtritt von Leitgel 6 durch die Defibrillierelektrode 4. Zum Zwecke der Durchlässigkeit für das Leitgel 6 kann die Defibrillierelektrode 4 porös sein. Im dargestellten und beschriebenen Ausführungsbeispiel der Erfindung weist sie durch Laserbearbeitung hergestellte Schlitze 7 auf, deren Schlitzwandungen in drucklosem Zustand aneinander anliegen und die sich bei Druckbeaufschlagung öffnen und dadurch einen Durchtritt des Leitgels 6 ermöglichen.

Die Löcher 2 aufweisende Struktur 3 ist eine Wabenmatte aus flexiblem Kunststoff, deren Stege in der Zeichnung mit der Bezugszahl 8 bezeichnet sind und deren Kammem die Löcher 2 bilden. In den Löchern 2 ist das Leitgel 6 enthalten, die Waben oder Löcher 2 der Löcher 2 aufweisenden Struktur 3 bzw. die Löcher 2 aufweisende Struktur 3 bilden ein das Leitgel 6 enthaltendes Reservoir 9. An Stelle der Wabenmatte kann beispielsweise auch ein offenporiger Schaum oder ähnliches die die Löcher 2 aufweisende Struktur 3, d, h. das Reservoir 9, bilden (nicht dargestellt).

Die Leiterplatte 5 weist ein flexibles Substrat mit in herkömmlicher Weise hergestellten, beispielsweise geätzten Leiterbahnen auf. In den Löchern 2 der Struktur 3 weist die Leiterplatte 5 elektrische Widerstände als Heizungen 10 auf. Zur Bildung der elektrischen Widerstände können die Leiterbahnen schmal sein oder es ist ein Widerstandsmaterial, beispielsweise durch Graphit-Pastendruck, aufgebracht. In den Löchern 2, die das Reservoir 9 bilden, ist die Leiterplatte 5 mit einer Chemikalie oder einem Chemikaliengemisch 11 überdeckt, das die Heizung 10 überdeckt und das bei Erwärmung chemisch reagiert und ein Gas bildet. Die Reaktion kann exotherm oder unter ständiger Wärmezufuhr endotherm ablaufen. Die Chemikalie oder das Chemikaliengemisch 11 ist ein Feststoff. Auch ist es möglich, dass die Chemikalie oder das Chemikaliengemisch im Leitgel oder auch in einem zweiten Gel oder einer Flüssigkeit enthalten ist. Mit der Heizung 10 bildet sie einen Gaserzeuger und eine Druckerzeugungseinrichtung, die nachfolgend als Aktivierungseinrichtung 12 bezeichnet wird.

Die in der Zeichnung mit t₁ bis t₄ bezeichneten Löcher 2 zeigen bei der Aktivierung der Defibrillierelektrode 1 zeitlich aufeinander folgende Zustände der Löcher 2, die von allen Löchern 2 gleichzeitig erreicht werden. Es weisen alle Löcher 2 den gleichen Zustand und die gleiche Füllung auf, die Darstellung aufeinander folgender Zeitpunkte t₁ bis t₄ dient der Erläuterung der Funktion der erfindungsgemäßen Defibrillierelektrode 1.

Zum Schutz vor Austrocknung des Leitgels 6 kann eine Gas-, insbesondere eine Dampfblase auf der Seite der eigentlichen Defibrillierelektrode 4 in den Löchern 2 enthalten sein, die in der Zeichnung mit der Strichlinie 13 angedeutet ist. Die Gas-, insbesondere die Dampfblase 13 verkleinert einen Dampfdruckgradienten vom Loch 2 zur Außenseite der Defibrillierelektrode 4 und verringert dadurch eine Austrocknung des Leitgels 6 auf einen Bruchteil, sofern die Durchlässe 7 die Löcher 2 nicht dicht verschließen.

Zur Verwendung wird die Defibrillierelektrode 1 mit der eigentlichen Defibrillierelektrode 4 im Brustbereich auf die Haut eines Patienten aufgesetzt und befestigt. Mit der elektrisch leitenden Defibrillierelektrode 4 werden die Herzströme gemessen bzw. die Herztätigkeit überwacht. Im Falle eines Herzstillstands oder eines Herzkammerflimmerns wird zur Aktivierung die Druck- oder Gaserzeugungseinrichtung der Aktivierungseinrichtung 12 durch Bestromen der Heizung 10 aktiviert. Die Wärme der Heizung 10 setzt die chemische Reaktion der Chemikalie oder des Chemikaliengemischs 11 in Gang (Zeitpunkt t₂). Es wird Gas gebildet (Zeitpunkt t₃) und es entsteht eine sich vergrößernde Gasblase 14 (Zeitpunkt t₄). Der dadurch bewirkte, auf das Leitgel 6 in den das Reservoir bildenden Löchern 2 ausgeübte Druck öffnet die Durchlässe bildendend Schlitze 7 in der eigentlichen Defibrillierelektrode 4 und verdrängt das Leitgel 6 durch die die Durchlässe bildenden Schlitze 7 aus den Löchern 2. Das Leitgel 6 tritt wie zum Zeitpunkt t₄ gezeigt auf der hautzugewandten Seite aus der Defibrillierelektrode 1, 4 aus und verteilt sich zwischen der Haut des Patienten und der Defibrillierelektrode 1, 4. Dadurch wird der Übergangswiderstand zwischen der Defibrillierelektrode 1, 4 und der Haut herabgesetzt und die Defibrillation durch Stromschock kann erfolgen. Der Stromschock erfolgt über die elektrisch leitende, eigentliche Defibrillierelektrode 4, die auch zum Messen bzw.

Überwachen der Herztätigkeit verwendet wird. Die Defibrillation kann mit steigenden Stromstärken wiederholt werden, wenn der Herzschlag nicht wieder einsetzt.

Die Dicke der dargestellten Defibrillierelektrode 1 beträgt etwa 1 bis 2 mm, ihre Fläche beträgt zwischen etwa 1 cm² bis zu einem Durchmesser von etwa 3 bis 5 cm (7 bis 20 cm²).

## Patentansprüche

1. Defibrillierelektrode zum Tragen auf der Haut, **dadurch gekennzeichnet, dass** die Defibrillierelektrode (4) bei einer Aktivierung durchlässig für ein Leitmedium (6) ist, dass die Defibrillierelektrode (4) ein das Leitmedium (6) enthaltendes Reservoir (2, 3) auf einer hautabgewandten Seite aufweist und dass die Defibrillierelektrode (4) eine Einrichtung (12) zum Durchdrücken des Leitmediums (6) durch die Defibrillierelektrode (4) zur Aktivierung der Defibrillelektrode (4) (Aktivierungseinrichtung (12)) aufweist.

2. Defibrillierelektrode nach Anspruch 1, **dadurch gekennzeichnet, dass** die Aktivierungseinrichtung (12) eine Druckerzeugungseinrichtung aufweist, mit der im Reservoir (2, 3) ein Druck zum Durchdrücken des Leitmediums (6) durch die Defibrillierelektrode (4) zur Aktivierung der Defibrillierelektrode (4) aufweist.

3. Defibrillierelektrode nach Anspruch 1 oder 2, **dadurch gekennzeichnet, dass** die Aktivierungseinrichtung (12) einen Gaserzeuger aufweist.

4. Defibrillierelektrode nach Anspruch 3, **dadurch gekennzeichnet, dass** eine Gaserzeugung durch chemische Reaktion erfolgt.

5. Defibrillierelektrode nach Anspruch 3, **dadurch gekennzeichnet, dass** der Gaserzeuger (12) eine Heizung (10) oder Zündung aufweist.

6. Defibrillierelektrode nach Anspruch 3, **dadurch gekennzeichnet, dass** eine Gaserzeugung elektrolytisch erfolgt.

7. Defibrillierelektrode nach Anspruch 2, **dadurch gekennzeichnet, dass** die Aktivierungseinrichtung (12) einen Dampferzeuger aufweist.

8. Defibrillierelektrode nach einem der vorhergehenden Ansprüche, **dadurch gekennzeichnet, dass** die Defibrillierelektrode (4) eine Löcher (2) aufweisende Struktur (3) als Reservoir auf einer hautabgewandten Seite aufweist.

9. Defibrillierelektrode nach einem der vorhergehenden Ansprüche, **dadurch gekennzeichnet, dass** die Defibrillierelektrode (4) porös ist.

10. Defibrillierelektrode nach einem der Ansprüche 1 bis 8, **dadurch gekennzeichnet, dass** die Defibrillierelektrode (4) Durchlässe (7) für das Leitmedium (6) aufweist.

11. Defibrillierelektrode nach einem der vorhergehenden Ansprüche, **dadurch gekennzeichnet, dass** das Reservoir (2) eine Gasblase (13) auf der Seite der Defibrillierelektrode (4) aufweist.

12. Defibrillierelektrode nach einem der vorhergehenden Ansprüche, **dadurch gekennzeichnet, dass** die Defibrillierelektrode (4) eine Leiterplatte (5) aufweist und dass zwischen der Leiterplatte (5) und der Defibrillierelektrode (4) das Reservoir (2) angeordnet ist.

13. Defibrillierelektrode nach einem der vorhergehenden Ansprüche, **dadurch gekennzeichnet, dass** die Defibrillierelektrode (1, 4) flexibel ist.
